# EUROPEAN PATENT APPLICATION

(11) **EP 3 326 629 A1**
(43) Date of publication of application: **30.05.2018**
(21) Application number: 16306562.6
(22) Date of filing: 25.11.2016
(51) Int. Cl.: A61K 31/4152, A61K 31/429, A61K 31/655, A61K 45/06, A61P 33/02, A61K 31/51, A61K 31/714

(54) **VETERINARY COMPOSITIONS AND THE USES THEREOF FOR PREVENTING AND/OR TREATING PARASITIC INFECTIONS IN NON-HUMAN MAMMALS**

(71) Applicant: Ceva Santé Animale, 33500 Libourne (FR)
(72) Inventor: KAREMBE, Hamadi, 33500 LIBOURNE (FR); LACOSTE, Sandrine, 33500 LIBOURNE (FR)
(74) Representative: Cabinet Becker et Associés

(57) **Abstract**

The present invention relates to a veterinary composition comprising diminazene and levamisole or salts thereof, wherein the weight ratio of diminazene or its salts to levamisole or its salts ranges from 0.10 to 5.00, preferably from 0.50 to 3.00, more preferably from 0.50 to 2.00. The present invention also relates to such veterinary composition for use for preventing and/or treating a parasitic infestation in a non-human mammal, preferably a bovine.

## Description

### FIELD OF THE INVENTION

The present invention relates to combinations or associations of active ingredients in the field of parasitology and veterinary compositions comprising such combinations, more particularly, for treating and/or preventing parasitic infestations such as trypanosomiasis and helminthiasis or other gastro-intestinal parasite infestations in non-human mammals.

### BACKGROUND OF THE INVENTION

Parasitic infestations have a major economic impact on cattle production and, if untreated, generally results in a chronic illness with high mortality. For instance, Animal African Trypanosomosis (AAT), caused principally by *Trypanosoma congolense* and *Trypanosoma vivax,* is estimated to cost African livestock farmers from 2 to 5 billon US dollar per year.

Most cases of trypanosomosis are chronic, but acute disease, which may be fatal within a week, can also occur. The first sign of trypanosomosis may be a localized swelling at the site of the fly bite. The primary clinical signs are an intermittent fever, signs of anemia, lymphadenopathy and weight loss. Animals lose condition and become progressively emaciated. Milk yield may be decreased in dairy animals and neurological signs, dependent edema, cardiac lesions, diarrhea, keratitis, lacrimation, appetite loss and other clinical signs have also been reported. Trypanosomosis also involve effects on reproduction such as abortions, premature births, and perinatal losses, as well as a testicular damage in males. Deaths are common among chronically infected animals and animals that recover clinically may relapse when stressed.

More generally, trypanosomes cause immunosuppression and concurrent infestations may complicate the disease due to a trypanosome infestation. For instance, mixed AAT and infestations by gastro-intestinal worms are common and occur mainly during the rainy season. In this context, diminazene/levamisole treatment with a potential parasiticide effect for both trypanosomes and gastro-intestinal parasites has been investigated.

Eghianruwa and Anika (International Journal of Animal and Veterinary Advances 4(5): 306-311, 2012) published a study on the efficacy of diminazene aceturate (known for its trypanocidal activity), and levamisole (a medication currently used to treat parasitic worm infestations) in reducing organ weight and parasitemia in *T. congolense* infected rats. In this study, the rats were intramuscularly treated with diminazene diaceturate at 7.0 mg/kg body weight seven days after *T. congolense* infection and 10,20, or 40 mg/kg body weight levamisole was administered as a daily supplement beginning from *T. congolense* infection. However, the results have shown a negative or deleterious effect of levamisole on diminazene since it has been observed that a treatment with diminazene followed by levamisole administration was less effective than diminazene alone in reducing spleen weight and that parasites disappeared after diminazene treatment but reappeared only in the animals treated with diminazene followed by levamisole. Such results confirmed the previous observations of Libeau and Pinder (Rev. Elev. Méd. Vet. Pays trop., 1981, 34 (4), 399-404) and Bisalla et al. (African Journal of Biotechnology Vol. 8 (5) 827-834, 2009) demonstrating the negative impact of levamisole in the treatment of *Trypanosoma congolense* infection in mice and sheep, respectively.

There is thus no efficient treatment known so far having a therapeutic efficacy against both trypanosomes and gastro-intestinal parasites in order to address these two infestations. Also, previous studies on diminazene and levamisole implemented in rats, mice, and sheep, have discouraged veterinary companies to use levamisole for treating trypanosome in a non-human mammal.

### SUMMARY OF THE INVENTION

Contrary to the above postulate, the inventors surprisingly provide a veterinary composition comprising diminazene and levamisole in a specific weight ratio, which is useful and efficient for treating a parasitic infestation in a non-human mammal, preferably both Trypanosoma infestation and gastro-intestinal infestation in a bovine. More particularly, the inventors have demonstrated that a composition as disclosed herein could restore body temperature and body weight, suppress parasitemia, and treat anemia in bovine infested with *Trypanosoma congolense* and/or gastro-intestinal and respiratory parasites.

The present invention therefore relates to a veterinary composition comprising diminazene and levamisole or salts thereof, wherein the weight ratio of diminazene or its salts to levamisole or its salts ranges from 0.10 to 5.00, preferably from 0.50 to 3.00, more preferably from 0.50 to 2.00. Even more preferably, such ratio ranges from 0.50 to 1.00, advantageously from 0.60 to 0.80, more advantageously about 0.70.
In a particular embodiment, the veterinary composition comprises:
- from 5 to 50%, preferably from 10 to 30%, more preferably from 15 to 25%, by weight of diminazene or its salts; and
- from 10 to 50%, preferably from 20 to 40%, more preferably from 25 to 40%, by weight of levamisole or its salts;
relative to the total weight of the composition.
In a further particular embodiment, the veterinary composition comprises phenazone. Preferably, the veterinary composition comprises:
- from 15 to 25%, preferably from 20 to 25% by weight of diminazene or its salts;
- from 25 to 40%, preferably from 30 to 40% by weight of levamisole or its salts; and
- from 30 to 50%, preferably from 35 to 45%, more preferably about 40% by weight of phenazone;
relative to the total weight of the composition.
In a further particular embodiment, the veterinary composition comprises an additional active agent, preferably an antibiotic, an anti-inflammatory agent, an anthelmintic, an endectocide, a mineral or a vitamin, more preferably a vitamin, even more preferably vitamin B1 to B12, advantageously vitamin B12a.
In a preferred embodiment, the veterinary composition as disclosed herein is a powder. In a further preferred embodiment, the veterinary composition as disclosed herein is an injectable solution.

The present invention also relates to a veterinary composition as disclosed herein, for use for preventing and/or treating a parasitic infestation in a non-human mammal. Particularly, the parasitic infestation is an infestation with *Trypanosoma spp,* preferably *Trypanosoma congolense, Trypanosoma vivax, Trypanosoma brucei,* and/or *Trypanosoma evansi;* and/or with Babesia spp, preferably with *Babesia bovis, Babesia bigemina, Babesia divergens,* and/or *Babesia major;* and/or an infestation with gastro-intestinal and respiratory parasites, preferably with *Ostertagia spp, Haemonchus spp, Trichostrongylus spp, Cooperia spp, Nematodirus spp, Bunostomum spp, Oesophagostomum spp,* and/or *Dictyocaulus spp.*
More particularly, the non-human mammal is a bovine.
In a particular embodiment, the veterinary composition for use, as disclosed herein, is administered by intramuscular or subcutaneous injection, preferably by intramuscular injection, more preferably in a single take.

A further object of the present invention, is a kit comprising (a) one compartment comprising diminazene or its salts and (b) a second compartment comprising levamisole or its salts, wherein the weight ratio of diminazene or its salts to levamisole or its salts ranges from 0.10 to 5.00, preferably from 0.50 to 1.00, more preferably about 0.70, as a combined preparation for simultaneous, separate or sequential use for preventing and/or treating a parasitic infestation in a bovine.
Particularly, the kit as disclosed herein further comprises a package leaflet or user instructions including the information that said preparation is to be used for preventing and/or treating a parasitic infestation, preferably infestation with *Trypanosoma spp* and/or *Babesia spp,* and/or gastro-intestinal and respiratory parasite in a bovine.

### LEGEND TO THE FIGURES

**Figure 1****:** Variation of the body temperature in treated groups G2 and G3 and non-treated group G1 (G1: Control; G2: Diminazene diaceturate 3.5 mg/kg; G3: Diminazene diaceturate 3.5 mg/kg and Levamisole 5.0 mg/kg).
**Figure 2****:** Variation of the body weight in treated groups G2 and G3 and non-treated groups G1 (G1: Control; G2: Diminazene diaceturate 3.5 mg/kg; G3: Diminazene diaceturate 3.5 mg/kg and Levamisole 5.0 mg/kg).
**Figure 3****:** Variation of the hematocrit rate in treated groups G2 (Figure 3B) and G3 (Figure 3C) and non-treated group G1 (Figure 3A) (G1: Control; G2: Diminazene diaceturate 3.5 mg/kg; G3: Diminazene diaceturate 3.5 mg/kg and Levamisole 5.0 mg/kg).

### DETAILED DESCRIPTION OF THE INVENTION

### Composition

The present invention herein provides a veterinary composition comprising diminazene and levamisole or salts thereof, wherein the weight ratio of diminazene or its salts to levamisole or its salts ranges from 0.10 to 5.00, preferably from 0.50 to 3.00, more preferably from 0.50 to 2.00. In a preferred embodiment, the weight ratio of diminazene or its salts to levamisole or its salts ranges from 0.50 to 1.00, preferably from 0.60 to 0.80, more preferably about 0.70.
As disclosed herein, weight ratios are calculated on the basis of the present diminazene or its salts, for instance diminazene diaceturate 4H₂O salt.

The present invention also provides a veterinary composition comprising:
- from 5 to 50%, preferably from 10 to 30%, more preferably from 15 to 25%, by weight of diminazene or its salts; and
- from 10 to 50%, preferably from 20 to 40%, more preferably from 25 to 40%, by weight of levamisole or its salts;
relative to the total weight of the composition.

Diminazene (also called 4-[2-(4-carbamimidoylphenyl)iminohydrazinyl] benzenecarboximidamide), is a di-amidine derivative having the following formula:

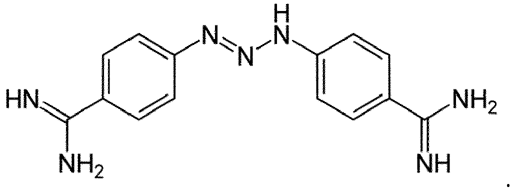

Diminazene is currently used as anti-infective drug and is effective against protozoa such as Babesia, Trypanosoma, and Cytauxzoon.

Levamisole (also called (*S*)-6-phenyl-2,3,5,6-tetrahydroimidazo[2,1-b][1,3]thiazole) has the following formula: 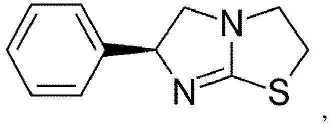 and was discovered in 1966 by Janssen Pharmaceutica. Levamisole is a drug used to treat parasitic worm infestations, particularly as a dewormer in veterinary medicine. However and as reported in the introduction part, levamisole is known to have deleterious or negative effect for treating Trypanosoma infestations.

Within the context of the invention, the salts of the active compounds of the combination of the invention or the compositions comprising such combination correspond to its pharmaceutically acceptable salts. A pharmaceutically acceptable salt includes inorganic as well as organic acids salts. Representative examples of suitable inorganic acids include hydrochloric, hydrobromic, hydroiodic, phosphoric and the like. For instance, a preferred salt of levamisole is levamisole hydrochloride. Representative examples of suitable organic acids include formic, acetic, trichloroacetic, propionic, benzoic, cinnamic, fumaric, maleic, methanesulfonic and the like. For instance, a preferred salt of diminazene is diminazene diaceturate.

As used herein, the term "about" will be understood by a person of ordinary skill in the art and will vary to some extent on the context in which it is used. If there are uses of the term which are not clear to persons of ordinary skill in the art given the context in which it is used, "about" will mean up to plus or minus 20 %, preferably 10 % of the particular term.

In a preferred embodiment, the veterinary composition further comprises phenazone. Phenazone (also called 1,2-dihydro-1,5-dimethyl-2-phenyl-3*H*-pyrazol-3-one) of the following formula: 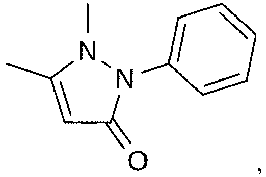 is an analgesic, a non-steroidal anti-inflammatory drug and an antipyretic, currently used to relieve pain and fever.
In this preferred embodiment, phenazone stabilizes the compositions of the invention. Indeed, the inventors have demonstrated that compositions comprising a weight ratio phenazone/diminazene or its salts superior to 1.6 avoid any precipitation and allow to maintain the solution as a clear yellow solution after 24 hours storage at room temperature.
In a more preferred embodiment, the veterinary composition further comprises phenazone, in which the weight ratio of phenazone to diminazene or its salts is greater than 1.6, preferably between 1.6 and 4.8, more preferably between 1.6 and 2.0, even more preferably between 1.7 and 1.9.

The veterinary compositions of the invention optionally comprises an additional active agent such as an antibiotic, an anti-inflammatory agent, an anthelmintic, an endectocide, a mineral, or a vitamin. In a preferred embodiment, the additional active agent is a vitamin, preferably vitamin B1 to B12, more preferably vitamin B12a (cyanocobalamin) or vitamin B12b (hydroxocobalamin).

The veterinary composition of the invention may also comprise acceptable excipients or carriers such as hydrophilic solvents. Such solvents are semi-polar to polar solvents and are typically used in granulation processes. For instance, hydrophilic solvents include without limitation water, alcohols such as methanol, ethanol, propanol, butanol, and glycerol, and ethers such as diethyl ether, polyethylene glycol, propylene glycol, and diethylene glycol monoethyl ether. In a preferred embodiment, the veterinary composition of the invention comprises ethanol.

In a particular embodiment, the composition as disclosed herein is a powder. This powder can be prepared according to a process comprising the steps of mixing solid active ingredients to form a blend which is then granulated by continuously adding a hydrophilic solvent such as ethanol, and finally dried to form the powder.

A preferred composition of the invention is thus a veterinary composition, preferably in a powder form, comprising:
- from 15 to 25%, preferably from 20 to 25% by weight of diminazene or its salts;
- from 25 to 40%, preferably from 30 to 40% by weight of levamisole or its salts;
- from 30 to 50%, preferably from 35 to 45%, more preferably about 40% by weight of phenazone;
relative to the total weight of the composition.

In a further preferred embodiment, the veterinary composition, preferably in a powder form, comprises:
- between 1.10 and 1.20 g of diminazene or its salts, preferably about 1.16 g of diminazene diaceturate salt,
- between 1.50 and 2.00 g, levamisole or its salts, preferably about 1.75 g of levamisole hydrochloride salt, and
- between 1.90 and 2.30 g, preferably about 1.95 g of phenazone.

In the veterinary compositions as disclosed herein, the ingredients as above detailed are present in the same composition.

### Application

The present invention also provides a veterinary composition as disclosed herein for use for preventing and/or treating a parasitic infestation in a non-human mammal.
Another object of the invention is a use of a veterinary composition as disclosed herein for the manufacture of a drug for preventing and/or treating a parasitic infestation in a non-human mammal.
A further objet of the invention is a method for preventing and/or treating a parasitic infestation in a non-human mammal comprising administering an effective amount or an effective dose of a veterinary composition as disclosed herein in a non-human mammal in need thereof.

Within the context of the invention, the expression "effective dose" or "effective amount" means the dose or the quantity sufficient for preventing and/or treating a parasitic infestation. In a particular embodiment, the expression "effective dose" or "effective amount" allows to kill at least 50%, 60%, 70%, 80%, 90% and preferably 100% of the parasites infesting or having infested the non-human mammal.

As used herein, the term "treatment" or "treating" refers to any act intended to ameliorate the health status of said non-human mammal such as therapy, prevention, prophylaxis and retardation of a parasitic infestation as disclosed herein. In certain embodiments, such term refers to the amelioration or eradication of a disease due to a parasitic infestation or symptoms associated with such disease. In other embodiments, this term refers to minimizing the spread or worsening of the disease due to the parasitic infestation resulting from the administration of compositions as disclosed herein to an infested non-human. The term "treatment" or "treating" also includes the preventive treatment of a non-human mammal against a disease due to a parasite infestation, which designates a treatment performed before the non-human mammal has been exposed to or in contact with the causative agent of the disease such as a parasite, or after said exposure/contact but before development of the symptoms or at an early stage of development of the disease due to a parasite infestation.

In a particular embodiment, the term "treatment" or "treating" designates the protection of a non-human mammal against a disease, e.g., against the effect of an exposure to the causative agent, or against the development of the disease in exposed non-human mammals. The invention particularly suits to protect non-human mammals against an infectious disease due to a parasitic infection or infestation. An object of the invention is therefore a method for protecting a non-human mammal against at least one parasitic infestation, comprising administering an effective amount or an effective dose of a veterinary composition as disclosed herein in a non-human mammal in need thereof. Such term also includes an increase in the welfare of the treated non-human mammal, for instance in increasing appetite, the production of meat, milk, bodyweight, etc., and in reducing fever or restoring body temperature.
The term "treatment" or "treating" also includes the alleviation of the symptoms such as a decrease of body weight, fever, anemia, as well as a delay, reduction, or cure of an existing infestation.
A further object of the invention is a method for restoring body temperature and/or body weight, and/or supressing parasitemia, and/or treating anemia in a non-human mammal comprising administering an effective amount or an effective dose of a veterinary composition as disclosed herein in a non-human mammal in need thereof.
A further object of the invention is a use of a composition or a composition for use as disclosed herein for restoring body temperature and/or body weight, and/or supressing parasitemia, and/or treating anemia in a non-human mammal.

Within the context of the invention, a "non-human mammal" comprises without limitation, canidae, for instance dogs, foxes, coyotes, and wolfs, felidae, for instance cats, lions, and panthers, bovidae, for instance cattle and dairy cow, equidae, for instance horses, and camelidae, for instance camel. In a preferred embodiment of the invention, a non-human mammal is cattle or a bovine.

Particularly, the veterinary composition as disclosed herein are used for treating a parasitic infestation such as trypanosomosis, babesiosis and/or gastro-intestinal parasites, preferably a parasitic infestation with *Trypanosoma spp* and/or *Babesia spp* and/or with gastro-intestinal and respiratory parasites, in a non-human mammal as above defined. Trypanosomes can infect all domesticated animals and clinical cases have been reported in cattle, water buffalo, sheep, goats, camels, horses, donkeys, alpacas, llamas, pigs, dogs, cats and other species. Trypanosomes include different species such as *Trypanosoma brucei, T. vivax, T. simiae, T. gambiense, T. rhodesiense, T. congolense, T. cruzi, T. evansi,* or *T. equinum.* The host preferences of each trypanosome species may differ but *Trypanosoma congolense, T. vivax, T. brucei,* and *T. simiae* have a wide host range among domesticated animals such as pigs, camels, horses and cattle. In a particular embodiment, a Trypanosoma infestation according to the invention results from an invasion of *Trypanosoma congolense, Trypanosoma vivax, Trypanosoma brucei,* and/or *Trypanosoma evansi, preferably Trypanosoma congolense.*

*Babesia* spp can also infect all domestic animals and cause babesiosis), toxoplasmosis and cryptosporidiosis. Particularly in bovines, it causes hemolytic anemia. As an example of *Babesia* spp, *B. duncani, B. divergens, B. venatorum, B. microti, B. duncan, B. bovis, B. bigemina, and B. major* may be cited without limitation. In a particular embodiment, a Babesia infestation according to the invention results from an invasion of *Babesia bovis, Babesia bigemina, Babesia divergens,* and/or *Babesia major.*

Gastro-intestinal parasites infect the gastro-intestinal tract of human and non-human mammals. They can live throughout the body but most preferably within the gastro-intestinal tract. Respiratory parasites infects airways and lungs of humans and non-human mammals. Gastro-intestinal and respiratory parasites include without limitation helminths and protozoa. In a preferred embodiment, gastro-intestinal and respiratory parasites are nematodes and are, more particularly, *Ostertagia spp, Haemonchus spp, Trichostrongylus spp, Cooperia spp, Nematodirus spp, Bunostomum spp, Oesophagostomum spp, and Dictyocaulus spp.* In a further particular embodiment, a gastro-intestinal parasite infestation results from an invasion of *Ostertagia spp, Haemonchus spp, Trichostrongylus spp, Cooperia spp, Nematodirus spp, Bunostomum spp, Oesophagostomum spp, and*/*or Dictyocaulus spp.*

The veterinary compositions of the invention are thus suitable for regions where such parasites can be currently found such as Africa, Asia and South America. The compositions of the invention have thus the major advantage to exhibit parasiticide effect against both trypanosomes, gastro-intestinal, and respiratory parasites. A preferred object of the invention is therefore a veterinary composition as disclosed herein for use for treating and/or preventing an infestation with *Trypanosoma spp,* preferably *Trypanosoma congolense, Trypanosoma vivax, Trypanosoma brucei,* and/or *Trypanosoma evansi;* and/or with Babesia spp, preferably with *Babesia bovis, Babesia bigemina, Babesia divergens,* and/or *Babesia major;* and/or an infestation with gastro-intestinal and respiratory parasites, preferably with *Ostertagia spp, Haemonchus spp, Trichostrongylus spp, Cooperia spp, Nematodirus spp, Bunostomum spp, Oesophagostomum spp,* and/or *Dictyocaulus*

The compositions of the invention as disclosed herein can be administrated in a non-human mammal by any route known from a skilled person such as an oral route, a topical route or a parenteral route such as injection. In a preferred embodiment, the compositions of the invention are administered in a non-human mammal by intramuscular or subcutaneous injection, preferably by intramuscular injection. The results as illustrated by examples show that a single intramuscular injection is sufficient to prevent and/or treat a parasitic infestation in a non-human mammal. In a further preferred embodiment, the compositions of the invention are administered in a single take.

The compositions of the invention administered by intramuscular injection use techniques and/or devices known *per se* in the art. In this regard, intramuscular injection can be performed with a syringe, a gun, a micro-needle injection device, a needle-free injection device, a pulse device, etc. In a preferred embodiment, injection is performed with a needle injector or a syringe. In another particular embodiment, injection is performed with a needle-free injection device such as a pulse needle-free system, more particularly a spring-powered, battery-powered, or compressed-gas-powered device. Intramuscular injection may be made in any muscle. For livestock, such as cattle or bovines, intramuscular injection is preferably made in the area of the neck, or behind the ear, preferably as a deep intramuscular injection in the neck.

In this context, the composition of the invention is formulated as a solution or suspension, or any form suitable for intramuscular or subcutaneous injection. The composition may further comprise veterinary acceptable excipient(s) such as solvents, solubilisers, surfactants, antioxidants, thickeners, preservatives, anti-foaming agents, etc. Suitable solvents include, without limitation, physiologically acceptable water, alcohols, esters, vegetable oils, and the mixtures thereof, in isotonic solutions. Solubilisers include, for instance polyvinylpyrrolidone. Examples of suitable antioxidants include ascorbic acid, gallic acid esters, and sulphates. Suitable preservatives include, without limitation, benzyl alcohol, n-butanol, benzalkonium chloride, benzoic acid or citric acid. Anti-foaming agents include without limitation oil carrier such as mineral oil, vegetable oil, white oil or any other oil that is insoluble in the foaming medium, silicone oil, simethicone emulsion, wax and/or hydrophobic silica such as ethylene bis-stearamide (EBS), paraffinic waxes, ester waxes, silica, fatty alcohol, fatty acid soaps or esters, silicone compound, polyethylene glycol and polypropylene glycol copolymers and alkyl polyacrylates.

In a particular embodiment, the composition of the invention is an injectable solution. This solution can be prepared by adding water, preferably sterile water, to a veterinary composition in a powder form as above described.
For instance, an injectable solution of the invention comprises:
- about 1.16 g of diminazene or its salts,
- about 1.75 g of levamisole or its salts,
- about 1.95 g of phenazone,
said ingredients are dissolved in 20-30 mL of sterile water.

The dose of the active ingredients, diminazene and levamisole or salts thereof, may vary depending on the non-human animal species. Conventional dosages rates from 1 to 50 mg per kg bodyweight (mg/kg) of the non-human animal may be used, preferably from 1 to 20 mg/kg, more preferably from 1 to 10 mg/kg. A preferred dosage for an intramuscular injection in a bovine comprises about 3.5 mg/kg diminazene diaceturate and about 5.0 mg/kg levamisole.

Treatment can be applied as a unique injection or can be repeated several times, for instance every three weeks, once a month, once every two months or once every three months.

The invention further provides a kit comprising (a) one compartment comprising diminazene or its salts and (b) a second compartment comprising levamisole or its salts, wherein the weight ratio of diminazene or its salts to levamisole or its salts ranges from 0.10 to 5.00, preferably from 0.50 to 1.00, more preferably about 0.70, as a combined preparation for simultaneous, separate or sequential use for preventing and/or treating a parasitic infestation in a bovine.

As used herein, the terms "separate" or "sequential" mean that diminazene and levamisole are administered successively. For instance, diminazene is firstly administered in a bovine, and levamisole is secondly administered in the same non-human mammal, or vice versa. In this context, diminazene and levamisole are physically sufficiently distinct from being separately or sequentially administrable.

A further object of the invention is a kit as defined above, further comprising a package leaflet or user instructions including the information that said preparation is to be used for preventing and/or treating a parasitic infestation, preferably Trypanosoma infestation and/or gastro-intestinal and respiratory parasite infestation in a bovine.

Further aspects and advantages of the invention will be disclosed in the following experimental section. The examples below illustrate the present invention and are given as non-limiting illustrations.

### EXAMPLES

### Example 1: Compositions of the invention and the process of preparation thereof.

**Powder**

| **Ingredients** | % (**w**/**w**) |
|---|---|
| Diminazene diaceturate | 24 |
| Levamisole hydrochloride | 36 |
| Phenazone | 40 |

Diminazene diaceturate, levamisole hydrochloride, and phenazone have been 1 mm sieved and mixed during 2 minutes at 150 tr/min. The blending has been granulated by continuously adding anhydrous ethanol during 2 minutes, and then densified for 3 minutes. Granules have been dried during 4 hours at 37 °C in a stove, and then 1 mm sieved.

### Injectable solutions

The injectable solution of the invention (IVP 1) has been prepared by adding 26 mL of sterile water to 4.9 g of the powder composition as above defined.
The injectable solution IVP2 has been prepared by adding 15 mL of sterile water to 2.36 g Veriben^{®} (Diminazene).

### Example 2: Biological studies

### I. Materials and methods

The schedule of the study is illustrated in the table 1 below.

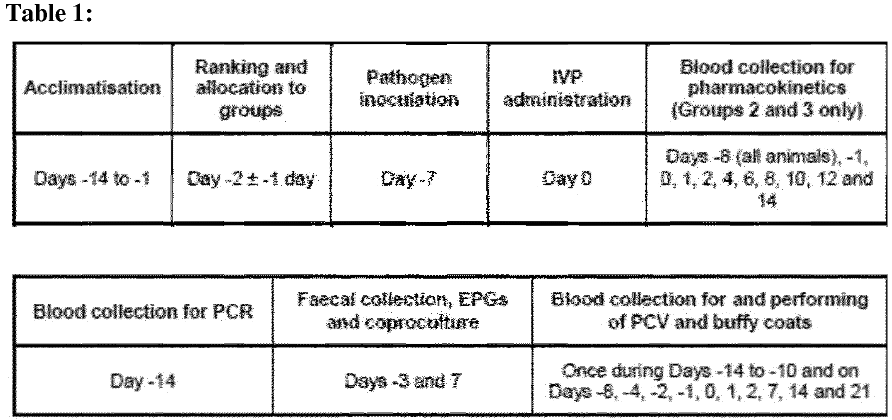

### 1. Acclimatisation (Days -14 to -1)

24 bovines (Nguni) were acclimimatised for at least 14 days before IVP (Investigational Veterinary Product) administration. No medication was given during the acclimatization period.

### 2. Ranking and allocation to groups (Day -2 ± -1 day)

Following clinical examination performed during acclimatisation period, 20 bovines were included and randomly allocated to the three groups (G1: n = 4; G2: n = 8; G3: n = 8).

### 3. Pathogen inoculation (Day -7)

Fresh cattle blood containing the pathogen T. congolense, 02J strain from donner cattle, was used to induce an infestation by intra venous inoculation on Day -7 for each bovine. Such pathogen was of a strain reported to be sensitive in cattle to diminazene.

### 4. IVP administration (Day 0)

The IVPs (Investigational Veterinary products) were administered by intra-muscular injection in the neck of the bovine once at day 0 at the dose rates indicated in table 2 below. 3.5 mg diminazene diaceturate per kg bodyweight corresponds to an injectable solution of 5.8 mL per 100 kg body weight (IVP2) for group 2. 3.5 mg diminazene diaceturate and 5.0 mg levamisole correspond to an injectable solution of 10 mL per 100 kg body weight (IVP1) for group 3. The animals in control group (Group 1) received a rescue treatment (Veriben^{®}) at Day 7.

**Table 2:**

| **Group** | **Sample size** | **IVP** | **Active ingredient(s)** | **Dose rate** |
|---|---|---|---|---|
| 1 | 4 | Not applicable | | |
| 2 | 8 | IVP2 (Veriben^{®}) | Diminazene | 3.5 mg diminazene diaceturate per kg bodyweight |
| 3 | 8 | IVP 1 (C753) | Diminazene and levamisole | 3.5 mg diminazene diaceturate and 5.0 mg levamisole per kg bodyweight |

### 5. Blood collection for PCR (Day -14)

Blood specimens of at least 3 mL were collected in EDTA tubes for PCR (Polymerase Chain Reaction) analysis once for each animal on Day -14. A strain specific PCR analysis of the pathogen strain used was also done prior to inoculation of donor animals to confirm the strain had not been contaminated by other *Trypanosoma* strains.

### 6. Faecal collection, EPGs and coproculture

Faecal egg counts were performed as a general screening to determine the possible presence of worm infections, if any. Faecal specimens were collected from each animals on Days -3 and 7. Animals were housed individually and fresh faeces were collected from the floor or collected from the rectum of the animal. Collected faeces were broken and mixed well for each animal, individually. A 4 g sample of faeces, taken from the well-mixed specimen, was suspended with the aid of a magnetic stirrer in 56 mL of a sucrose solution [sugar 400 g, H₂O 600 mL]. While stirring the suspension, a Pasteur pipette was used to fill the counting chambers of a MacMaster slide. The slide consisted of two persplex slides, separated by strips of Perspex 1.5 mm thick and with a 1.0 cm2 area ruled over each chamber. Eggs in the suspension floated upwards in the counting chamber and could then be counted under a microscope within the 1.0 cm2 area. Two or three chambers were counted depending on slide type used and the mean number calculated. Egg numbers were then expressed as eggs per gram of faeces (EPG) as follows:

### Mean number of eggs counted in the squares X 100

Coprocultures were performed as part of a general screening to determine the possible presence of worm infections, if any. Nematode species infecting bovines could not always be accurately identified on egg morphology alone. Faecal cultures were prepared, incubated and harvested as per study site SOP. Faces from individual animal were mixed with vermiculite to attain a moist granular consistency. Small amounts of bore-hole water were added when the mixture was too dry. The mixture was transferred to a suitable container and lightly compressed to form a cake in the bottom. It was compressed just sufficiently so that it did not fall out when the container was turned over and were not too wet. The inside of the container was wiped clean above the level of the compressed mixture. The container was covered in such a way that some air circulation was possible and marked with at least the date of collection and culture preparation, study number and animal ID. The prepared culture was incubated at approximately 27 °C. After approximately 3 days, the inside of the container was aerated by lifting the lid and sides wetted lightly if the content appeared too dry. Between 5 to 7 days, the containers could be removed from the incubator; lightly wetted and left outside the incubator. After approximately an hour, hatched larvae crawled up in the film of moisture on the inside of the container. They could be washed out with dechlorinated water. Larvae were stored in shallow water in a properly parked container at 8 °C to 10 °C. Each individual suspension was mixed to allow a homogeneous distribution of larvae. From each suspended solution, the first 100 larvae encountered were identified. Species composition was then expressed as a percentage to provide an indication of prevalence.

### 7. Blood collection for PCV and parasitemia/buffy coats

Blood specimens of approximately 3 mL for determination of packed cell volume (PCV) and parasitemia were collected for all animals in heparinized collection tubes from a nominated vein on Day -14 for enrolment and on Days -8, -4, -3, -2, -1, 0, 1, 2, 7, 14 and 21.

### II. Results

### 1. Local and general tolerance of a single intra-muscular injection of IVP 1 and UVP 2.

Intramuscular IVP1 and IVP2 were well tolerated. No pain for animal and local reaction (Edema) has been observed.

### 2. Body temperature.

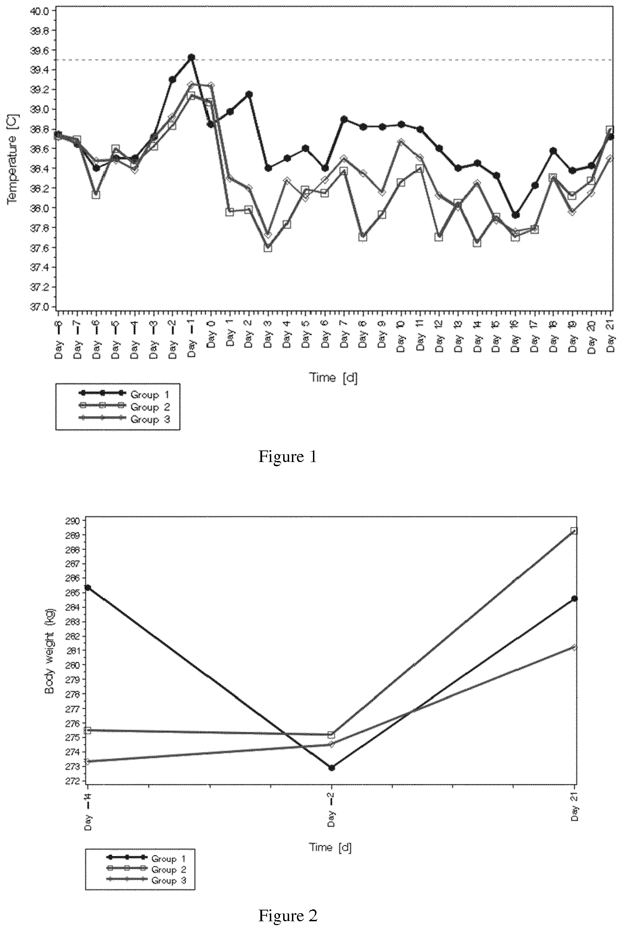

Body temperatures were measured on Days -14 and daily form Days -8 to Day 21. As reported by figure 1, control group 1 (infected and not treated) had the highest body temperatures on Day 0 and the body temperature both decreased for Group 2 and 3 after IVP administration, thereby demonstrating a fever reduction with IVP 1 and IVP 2 treatments.

### 3. Bodyweight.

The animals were weighted on a calibrated and verified electronic scale on Days -14, -2, and 21. As reported by figure 2, body weight gain has been observed after IVP 1 and IVP 2 administrations, thereby demonstrating a biological effect with IVP 1 and IVP 2 treatments. Body weight gain observed for control group is due to the rescue treatment with Veriben^{®}. On overall, the bodyweight development of animals in control group (Group 1) was lower.

### 4. Parasitemia (Trypanosoma congolense).

IVPs were fully effective in curing the animals. No parasite was observed in treated animals during the follow up period on Days 0, 1, 2, 7, 14, and 21 (Table 3). Fisher's exact test was used to compare the IVP groups and the control group together (Table 4). The difference between treated and control groups was highly significant. There was no significant difference between IVPs (IVP1 and IVP2).

**Table 3:**

| Summary of parasitaemia | | | | | |
|---|---|---|---|---|---|
| | Absent / N (%) | | | | |
| Group | Day 1 | Day 2 | Day 7 | Day 14 | Day 21 |
| Group 1 | 0/4 | 0/4 | 0/4 | 4/4 (100%) | 4/4 (100%) |
| Group 2 | 7/8 (87.5%) | 8/8 (100%) | 8/8 (100%) | 8/8 (100%) | 8/8 (100%) |
| Group 3 | 7/8 (87.5%) | 8/8 (100%) | 8/8 (100%) | 8/8 (100%) | 8/8 (100%) |

| | | | | | |
|---|---|---|---|---|---|
| Group 1: Negative control Group 2: Cattle received IVP 2, Veriben®, as a deep intra-muscular injection in the neck. Group 3: Cattle received IVP 1, C753, as a deep intra-muscular injection in the neck. | | | | | |

**Table 4:**

| Comparison | Day 1 | Day 2 | Day 7 |
|---|---|---|---|
| Group 2 - Group 1 | 0.0101 | 0.0020 | 0.0020 |
| Group 3 - Group 1 | 0.0101 | 0.0020 | 0.0020 |
| Group 3 - Group 2 | 1.0000 | | |

| | | | |
|---|---|---|---|
| p-value: fishers exact test Group 1: Negative control Group 2: Cattle received IVP 2, Veriben®, as a deep intra-muscular injection in the neck. Group 3: Cattle received IVP 1, C753, as a deep intra-muscular injection in the neck. | | | |

For the control group 1, all animals had parasitemia on Day 1, 2 and 7 and all animals had an absence of parasitemia on Days 14 and 21 after recue treatment administered on Day 8.
For both IVP groups (Groups 2 and 3), only one animal had parasitemia on Day 1 and 100% of animals were cured 2 days following treatment.
The number of animals with an absence of parasitemia in the IVP groups (Groups 2 and 3) compared to the control group 1 was statistically significant (p < 0.05). Also, comparison Group 3-Group 2 (p = 1.0000) has demonstrated that there was no negative effect due to the levamisole.

### 5. Trypanosoma PCV.

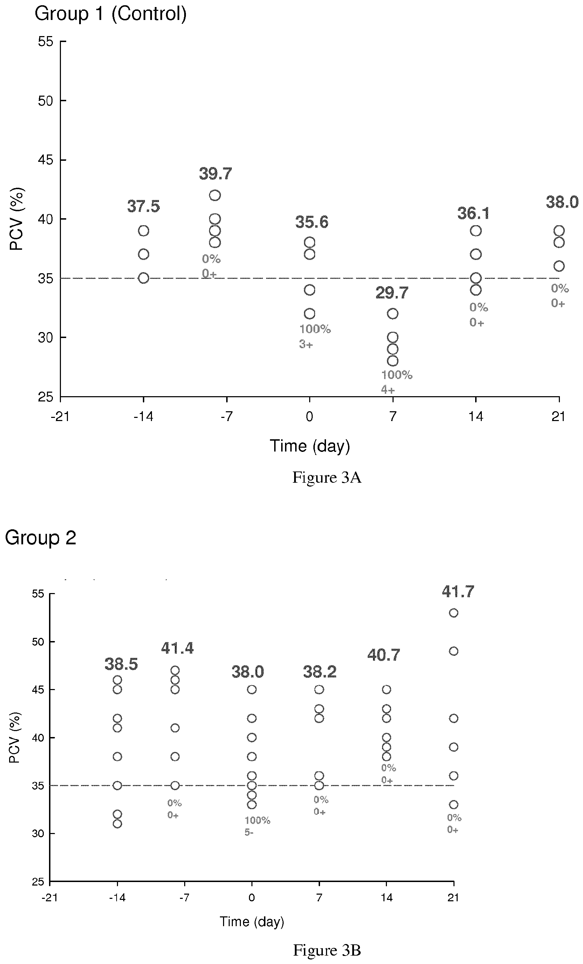

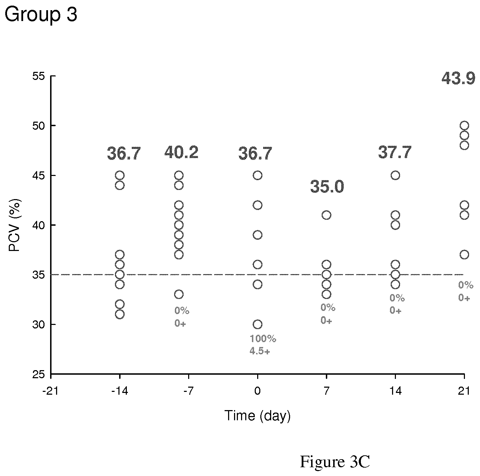

Mean PCV for each group for Days -14, -7, 0, 7,14, and 21 were calculated and compared using an ANOVA test. Results are reported in Figures 3A-3C. As it has been observed, the mean PCV values in the control group 1 (29.7% at D7) was less than the IVP groups (38.2% for Groups 2 and 35% for Group 3 at D7) after Day 0, thereby demonstrating full recovery of hematocrit levels (PCV) with IVP 1 and IVP 2 treatments. Of note that the Control group (Group 1) received a rescue treatment on Day 8, explaining the increase of the hematocrit on Days 14 and 21.

### 6. Faecal egg counts and coproculture.

Parasite Eggs per gram of faeces (EPC) for each group is summarized in Table 5 below.

**Table 5:**

| Group | Statistic | Day -3 | Day 7 |
|---|---|---|---|
| Group 1 | n | 4 | 4 |
| | Mean | 158.33 | 133.33 |
| | SD | 206.155 | 81.650 |
| | Median | 100.00 | 133.33 |
| | Minimum | 0.0 | 33.3 |
| | Maximum | 433.3 | 233.3 |
| | | | |
| Group 2 | n | 8 | 8 |
| | Mean | 95.83 | 41.67 |
| | SD | 114.694 | 46.291 |
| | Median | 33.33 | 33.33 |
| | Minimum | 0.0 | 0.0 |
| | Maximum | 300.0 | 133.3 |
| | | | |
| Group 3 | n | 8 | 8 |
| | Mean | 37.50 | 0.00 |
| | SD | 45.207 | 0.000 |
| | Median | 16.67 | 0.00 |
| | Minimum | 0.0 | 0.0 |
| | Maximum | 100.0 | 0.0 |

All animals in group 3 (IVP 1) had no eggs per gram, while group 1 (control) and group 2 (IVP 2) still had some eggs (133.33 and 4.291 per gram, respectively) after IVP administration on Day 0.

Larval identification and enumeration following coproculture is reported in the table 6 below. Harvesting of faecal cultures and subsequent enumeration and identification yielded two nematode species, namely *Ostertagia ostertagi* and *Haemonchus placei.* Coproculture results support the trend identified when using faecal egg counts, namely little reduction in worms number between Days -3 and 7 for groups 1 and 2 (Control and IVP 2 groups), with very few worms present in group 3 on Day 7. For group 3 prevalence decreased from 100% (all 8 animals infected with at least one of the worm species on Day -3) to 50% (4 animals infected with at least one of the worm species on Day 7). In addition, intensity of infection (total number of worms) decreased from 100 worms (100 larvae examined for identification purposes) to 2 worms between Day -3 and Day 7. The overall curative efficacy of the combination was 98%.

**Table 6:**

| Group | SD faeces collected | Animal ID | *Ostertagia ostertagi* | *Haemonchus placei* | Prevalence | Intensity |
|---|---|---|---|---|---|---|
| NA | -3 | CVB 440 | 88 | 12 | 100 | 100.0 |
| | | CVB 446 | 96 | 4 | | |
| | | CVB 457 | 83 | 17 | | |
| 1 | | CVB 436 | 100 | 0 | | |
| | | CVB 438 | 86 | 14 | | |
| | | CVB 448 | 84 | 16 | | |
| | | CVB 452 | 72 | 28 | | |
| | 7 | CVB 436 | 96 | 4 | 100 | 100.0 |
| | | CVB 438 | 96 | 4 | | |
| | | CVB 448 | 98 | 2 | | |
| | | CVB 452 | 94 | 6 | | |
| 2 | -3 | CVB 437 | 94 | 6 | 100 | 99.9 |
| | | CVB 445 | 77 | 23 | | |
| | | CVB 449 | 75 | 25 | | |
| | | CVB 451 | 74 | 26 | | |
| | | CVB 453 | 100 | 0 | | |
| | | CVB 454 | 89 | 10 | | |
| | | CVB 455 | 100 | 0 | | |
| | | CVB 456 | 83 | 17 | | |
| | 7 | CVB 437 | 97 | 3 | 100 | 100.0 |
| | | CVB 445 | 93 | 7 | | |
| | | CVB 449 | 81 | 19 | | |
| | | CVB 451 | 88 | 12 | | |
| | | CVB 453 | 99 | 1 | | |
| | | CVB 454 | 96 | 4 | | |
| | | CVB 455 | 100 | 0 | | |
| | | CVB 456 | 93 | 7 | | |
| 3 | -3 | CVB 441 | 100 | 0 | 100 | 100.0 |
| | | CVB 442 | 95 | 5 | | |
| | | CVB 443 | 94 | 6 | | |
| | | CVB 444 | 93 | 7 | | |
| | | CVB 447 | 93 | 7 | | |
| | | CVB 450 | 84 | 16 | | |
| | | CVB 458 | 84 | 16 | | |
| | | CVB 459 | 62 | 38 | | |
| | 7 | CVB 441 | 1 | 0 | 50 | 2.0 |
| | | CVB 442 | 0 | 0 | | |
| | | CVB 443 | 0 | 0 | | |
| | | CVB 444 | 1 | 0 | | |
| | | CVB 447 | 3 | 1 | | |
| | | CVB 450 | 0 | 0 | | |
| | | CVB 458 | 2 | 0 | | |
| | | CVB 459 | 0 | 0 | | |
| **Note:** Total number of worms (combined for both species) used for calculation of intensity Prevalence = (Number of animals infected/Total number of animals assessed) x 100 Intensity = Total number of parasites/Number of infected animals | | | | | | |

| | | | | | | |
|---|---|---|---|---|---|---|
| Group 1: Negative control Group 2: Cattle received IVP 2, Veriben^{®}, as a deep intra-muscular injection in the neck. Group 3: Cattle received IVP 1, C753, as a deep intra-muscular injection in the neck | | | | | | |

### 7. Conclusions

In conclusion, the inventors have demonstrated that a composition of the invention comprising diminazene and levamisole had a similar efficient activity against trypanosomes compared to the commercially available formulation Veriben^{®} (Diminazene) when applied in bovines, thereby showing no negative effect or negative interaction of levamisole on Diminazene.

In addition, the inventors have demonstrated an additional efficient activity against gastro-intestinal parasites, such as nematodes, with the compositions of the invention.

### Example 3: Stability studies

### I. Materials and methods

Powder formulations of the invention have been prepared by the process of example 1 and were dissolved in 21 ml of sterile water. The composition for one dose is detailed by the following table 7.

**Table 7:**

| **Composition for one dose** | **A** | **B** | **C** | **D** | **Reference Powder for injection** | **Reference Solution for injection** |
|---|---|---|---|---|---|---|
| **Diminazene Diacéturate 4H₂0 (g)** | 1.16 | 1.16 | 1.16 | 1.16 | 1.16 | 1.16 |
| **Levamisole HCl (g)** | 1.75 | 1.75 | 1.75 | 1.75 | 0 | 0 |
| **Phenazone (g)** | 1.20 | 1.65 | 1.95 | 2.21 | 1.31 | 5.6 |
| **Total one dose (g)** | 4.11 | 4.50 | 4.90 | 5.12 | 2.47 | 15 mL |
| **Ratio Phenazone/Diminazene diaceturate** | 1.0 | 1.4 | 1.7 | 1.9 | 1.1 | 4.8 |

### II. Results

Stability of compositions A, B, C, and D are presented in the following table 8. The appearance of the obtained solution were noted immediately after reconstitution and after 24 hours storage at room temperature.

**Table 8:**

| Composition for one dose | **A** | **B** | **C** | **D** |
|---|---|---|---|---|
| **Appearance of solution at T0** | Clear yellow solution | | | |
| **Appearance of solution after 24 hours storage at room temperature** | Precipitation | Precipitation | Clear yellow solution | Clear yellow solution |

As shown by table 8, precipitation of diminazene occurs if the weight ratio phenazone/ diminazene diaceturate 4H₂O is less than 1.6. Therefore, the weight ratio Phenazone/Diminazene 4H₂O must be superior to 1.6 to avoid any precipitation of the complex for solubilization in water when volume is comprised between 20 ml and 30 ml. This amount of phenazone in the current formulation is higher than the amounts known and used in existing diminazene-based powder formulation (ratio = 1.2). It is lower when compared to the commercial ready to use solutions for injection (Ratio = 4.8).

## Claims

1. A veterinary composition comprising diminazene and levamisole or salts thereof, wherein the weight ratio of diminazene or its salts to levamisole or its salts ranges from 0.10 to 5.00, preferably from 0.50 to 3.00, more preferably from 0.50 to 2.00.

2. The veterinary composition according to claim 1, wherein the weight ratio of diminazene or its salts to levamisole or its salts ranges from 0.50 to 1.00, preferably from 0.60 to 0.80, more preferably about 0.70.

3. The veterinary composition according to claim 1 or 2, comprising:
- from 5 to 50%, preferably from 10 to 30%, more preferably from 15 to 25%, by weight of diminazene or its salts; and
- from 10 to 50%, preferably from 20 to 40%, more preferably from 25 to 40%, by weight of levamisole or its salts;
relative to the total weight of the composition.

4. The veterinary composition according to any one of claims 1 to 3, further comprising phenazone.

5. The veterinary composition according to any one of claims 1 to 4, comprising:
- from 15 to 25%, preferably from 20 to 25% by weight of diminazene or its salts;
- from 25 to 40%, preferably from 30 to 40% by weight of levamisole or its salts; and
- from 30 to 50%, preferably from 35 to 45%, more preferably about 40% by weight of phenazone;
relative to the total weight of the composition.

6. The veterinary composition according to any one of claims 1 to 5, comprising an additional active agent, preferably an antibiotic, an anti-inflammatory agent, an anthelmintic, an endectocide, a mineral or a vitamin.

7. The veterinary composition according to claim 6, wherein the additional active agent is a vitamin, preferably vitamin B1 to B12, more preferably vitamin B12a.

8. The veterinary composition according to any one of claims 1 to 7, wherein said composition is a powder.

9. The veterinary composition according to any one of claims 1 to 7, wherein said composition is an injectable solution.

10. A veterinary composition according to any one of claims 1 to 9, for use for preventing and/or treating a parasitic infestation in a non-human mammal.

11. The veterinary composition for use according to claim 10, wherein the parasitic infestation is an infestation with *Trypanosoma spp,* preferably *Trypanosoma congolense, Trypanosoma vivax, Trypanosoma brucei,* and/or *Trypanosoma evansi;* and/or with Babesia spp, preferably with *Babesia bovis, Babesia bigemina, Babesia divergens,* and/or *Babesia major;* and/or an infestation with gastro-intestinal and respiratory parasites, preferably with *Ostertagia spp, Haemonchus spp, Trichostrongylus spp, Cooperia spp, Nematodirus spp, Bunostomum spp, Oesophagostomum spp,* and/or *Dictyocaulus spp.*

12. The veterinary composition for use according to claim 10, wherein the non-human animal is a bovine.

13. The veterinary composition for use according to any one of claims 10 to 12, wherein said composition is administered by intramuscular or subcutaneous injection, preferably by intramuscular injection, more preferably in a single take.

14. A kit comprising (a) one compartment comprising diminazene or its salts and (b) a second compartment comprising levamisole or its salts, wherein the weight ratio of diminazene or its salts to levamisole or its salts ranges from 0.10 to 5.00, preferably from 0.50 to 1.00, more preferably about 0.70, as a combined preparation for simultaneous, separate or sequential use for preventing and/or treating a parasitic infestation in a bovine.

15. The kit according to claim 14, wherein it further comprises a package leaflet or user instructions including the information that said preparation is to be used for preventing and/or treating a parasitic infestation, preferably infestation with *Trypanosoma spp* and/or *Babesia spp,* and/or gastro-intestinal and respiratory parasite infestation in a bovine.
